# EUROPÄISCHE PATENTSCHRIFT

(11) **EP 2 373 278 B1**
(45) Veröffentlichungstag und Bekanntmachung des Hinweises auf die Patenterteilung: **24.10.2012**
(21) Anmeldenummer: 09801415.2
(22) Anmeldetag: 23.12.2009
(51) Int. Cl.: A61K 8/02, A61K 8/34, A61K 8/37, A61K 8/39, A61K 8/60, A61K 8/92, A61Q 19/00

(54) **Fliessfähige Emulsionskonzentrate**
Free-flowing emulsion concentrates
Concentré d'émulsion fluidifiable

(30) Priorität: 08.01.2009 EP 09000134
(43) Veröffentlichungstag der Anmeldung: 12.10.2011
(73) Patentinhaber: Cognis IP Management GmbH, 40589 Düsseldorf (DE)
(72) Erfinder: STRAUSS, Gabriele, 40589 Düsseldorf (DE); KAWA, Rolf, 40789 Monheim (DE); STORK, Anja, 50733 Köln (DE); SCHULTE, Petra, 50733 Köln (DE)
(86) Internationale Anmeldenummer: PCT/EP2009/009252
(87) Internationale Veröffentlichungsnummer: WO 2010/078946

(56) Entgegenhaltungen:
- WO-A1-92/07543
- WO-A1-2006/000360
- WO-A1-2008/019773

## Beschreibung

### Gebiet der Erfindung

Die vorliegende Erfindung betrifft bei niedrigen Temperaturen fließfähige und pumpbare Emulsionskonzentrate mit guten sensorischen Eigenschaften und guter Lagerstabilität, die insbesondere für kosmetische Tücher geeignet sind, und bei denen auf den Einsatz von ethoxylierten Rohstoffen verzichtet werden kann.

### Stand der Technik

Emulsionskonzentrate des Marktes basieren in der Regel auf ethoxylierten Rohstoffen, was aus ökologischer Sicht kritisch gesehen wird.

Für EO-freie (d.h. frei von ethoxylierten Rohstoffen), hydrophile Emulsionskonzentrate ist eine Kombination bekannt, die auf wasserunlöslichen Ölkomponenten, Emulgatoren und Polyolen basiert und z. B. unter der Bezeichnung Emulgade® CPE (Handelsname) erhältlich ist. Nachteilig an diesen Emulsionskonzentraten sind ihr Viskositätsanstieg bei niedrigen Temperaturen, insbesondere bei Temperaturen von unter 15°C und ihre sensorischen Eigenschaften.

WO 2008/019773 A1 beschreibt Emulsionskonzentrate mit wasserunlöslichen Komponenten, hydrophilen nichtionischen Emulgatoren, lipophilen Coemulgatoren, Polyolen und Wasser. Dabei werden Emulgatoren auf Basis ethoxylierter Substanzen verwendet. Weiterhin wird eine Fließ- und Pumpfähigkeit der Emulsionskonzentrate bei Raumtemperatur angegeben, die vorzugsweise mit geringen Anteilen an ethoxylierten Substanzen/Rohstoffen herstellbar sind. Von besonderem Interesse war es, Emulsionskonzentrates zur Verfügung zu Stellen, die ohne ethoxylierte Substanzen herstellbar sind.

WO 2006/000360 beschreibt fließfähige, pumpbare Emulsionskonzentrate zur Herstellung kosmetischer oder pharmazeitischer Emulsionen, die (a) 3 - 5 Gew. % Laurylglucosid (b) 1 - 2 Gew. % Myristylglucosid (c) 5 - 7 Gew. % Polyglycerin-2-dipolyhydroxystearat (d) 4 - 8 Gew.-% C12-C24-Fettalkohole (e) 70 - 75 Gew. % Olkörper (f) 4 - 5 Gew. -% eines Hydrotrops ausgewählt aus den Diolen, Polyolen oder aus Glycerin und (g) 2 -10 Gew. % Wasser enthalten.

Aufgabe der vorliegenden Erfindung war es, Emulsionskonzentrate zur Verfügung zu stellen, die bei Temperaturen unterhalb 15°C fließfähig und pumpbar sind und gute sensorische Eigenschaften aufweisen. Als fließfähig und pumpbar werden erfindungsgemäß Emulsionskonzentrate bezeichnet, deren Viskosität bei 15°C unterhalb 30000 mPas, gemessen mit einem Brookfield Rotationsviskosimeter (Typ RVF, Spindel 5, 10 UpM, 23 °C), liegt.

Diese Emulsionskonzentrate können unverdünnt auf Substrate aufgebracht werden, werden aber normalerweise verdünnt. Im einfachsten Fall erfolgt die Verdünnung mit Wasser. Es ist eine weitere Aufgabe der Erfindung, Emulsionskonzentrate bereit zu stellen, die nach Verdünnung lagerstabil sind, was im Allgemeinen gewährleistet ist, wenn die Teilchengröße, gemessen mit einem Coulter LS, kleiner 100 nm ist und die Verdünnungen transparent sind und nicht separieren.

### Beschreibung der Erfindung

Überraschenderweise wurde gefunden, dass Emulsionskonzentrate der vorliegenden Erfindung diese Probleme lösen. Der Begriff Emulsionskonzentrat wird synonym zum Begriff Konzentrat verwendet.

Gegenstand der vorliegenden Erfindung ist ein Emulsionskonzentrat, enthaltend folgende Komponenten:
(A) Alkyl- und/oder Alkenyloligoglykosid(e) mit 6 bis 22 Kohlenstoffatomen im Alk(en)ylrest,
(B) Polyglycerotester,
(C) Polyol(e),
(D) Glycerid(e), die einen Schmelzpunkt größer gleich 20 °C aufweisen
(E) bei 20 °C flüssige Ölkomponente(n) mit einer Polarität von größer 30 - 40 mN/m und
(F) bei 20 °C flüssige Ölkomponente(n) mit einer Polarität von 5 - 30 mN/m,
wobei
- die Komponenten (A), (B) und (C) ein Gewichtsverhältnis A : B : C von 1 : (0,6 - 0,8) : (0,9 - 1,1) aufweisen,
- die Komponenten (A) und (D) ein Gewichtsverhältnis A : D von 4 : 1 bis 1,5 : 1 aufweisen,
- die Komponenten (E) und (F) ein Gewichtsverhältnis E : F von 1 : 1 bis 1 : 3 aufweisen, und
- der Wasseranteil kleiner gleich 20 Gew.-% bezogen auf das Gesamtgewicht des Emulsionskonzentrats beträgt.

Werden diese Bedingungen eingehalten, können Emulsionskonzentrate erhalten werden, welche bei 15°C eine Viskosität von unterhalb 30000 mPa*s aufweisen, bestimmt mittels eines Brookfield Rotationsviskosimeters (Typ RVF, Spindel 5, 10 UpM, 23 °C). Bevorzugt weisen die erfindungsgemäßen Emulsionskonzentrate bei 15°C eine Viskosität von unterhalb 20000 mPa*s, weiter bevorzugt unter 15000 mPa*s, auf.

Sowohl die erfindungsgemäßen Emulsionskonzentrate als auch deren Verdünnungen weisen eine gute Lagerstabilität auf, welche sich darin äußert, dass bspw. nach 5 %-iger Verdünnung mit Wasser nach einer Woche die Verdünnungen transparent sind und die mittlere Teilchengröße, bestimmt mittels eines Coulter LS, kleiner 100 nm ist.

Die vorliegende Erfindung betrifft auch kosmetische und/oder pharmazeutische Zubereitungen, welche durch Verdünnen des erfindungsgemäßen Emulsionskonzentrats erhalten werden.

Weiterhin betrifft die vorliegende Erfindung Substrate, insbesondere Papier-, Vlies- oder Gewebe-Produkte zur Körperpflege und/oder -reinigung, wie z. B. kosmetische Tücher, welche das Emulsionskonzentrat oder die kosmetische und/oder pharmazeutische Zubereitung enthalten.

Überraschenderweise wurde gefunden, dass Emulsionskonzentrate hergestellt werden können, die bei niedrigen Temperaturen fließfähig und pumpbar sind, gute sensorische Eigenschaften aufweisen und lagerstabil sind, ohne dass bei der Formulierung ethoxylierte Substanzen verwendet werden müssen. Diese Wirkungen können durch das Zusammenspiel der Komponenten und deren Mengenverhältnisse, wie in Anspruch 1 angegeben, überraschenderweise in dem Emulsionskonzentrat erzielt werden.

### Ölkomponente (Komponenten (E) und (F))

Ein wesentliches Merkmal der vorliegenden Erfindung ist die Zusammenstellung der lipophilen Phase hinsichtlich der Komponenten (E) und (F). Es ist erfindungsgemäß erforderlich, dass ein Teil der lipophilen Phase durch Komponenten mit niedriger Polarität gebildet wird (Komponente (F)) und ein Teil durch Komponenten mit hoher Polarität (Komponente (E)).

Dementsprechend weist erfindungsgemäß ein Teil der lipophilen Phase eine Polarität von 5 bis 30 mN/m (Komponente F) und ein anderer Teil eine Polarität von größer 30 - 40 mN/m auf (Komponente E).

Sowohl Komponente (E) als auch Komponente (F) sind bei 20 °C flüssig. Bei den Komponenten (E) und (F) handelt es sich definitionsgemäß um von den Komponenten (A), (B), (C) und (D) verschiedene Stoffe. Für die Berechnung der erfindungsgemäßen Verhältnisse der Komponenten zueinander, werden die Komponenten (A), (B), (C) und (D) nicht zu den Komponenten (E) und (F) gezählt, selbst wenn diese Komponenten eine Polarität im Bereich der für die Komponenten (E) und (F) angegebenen Werte aufweisen.

Die Polarität der Ölkomponenten (E) und (F) wird über die Grenzflächenspannung angegeben. Die Grenzflächenspannung ist die diejenige Kraft, die an einer gedachten, in der Grenzfläche zwischen zwei Phasen befindlichen Linie der Länge von einem Meter wirkt. Die physikalische Einheit für die Grenzflächenspannung errechnet sich klassisch nach der Beziehung Kraft/Länge und wird gewöhnlich in mN/m (Millinewton geteilt durch Meter) wiedergegeben. Sie hat ein positives Vorzeichen, wenn sie das Bestreben hat, die Grenztläche zu verkleinern, im umgekehrten Fall hat sie ein negatives Vorzeichen. Kleinere positive Werte bedeuten eine höhere Polarität. Die Grenzflächenspannung wird erfindungsgemäß nach der ASTM Methode D971-99a (reapproved 2004) bestimmt.

Es war nicht zu erwarten, dass durch die erfindungsgemäße Polaritätenverteilung in der lipophilen Phase die Fließfähigkeit der Emulsionskonzentrate bei niedrigen Temperaturen verbessert werden konnte und gleichzeitig andere Vorteile erzielt werden konnten, wie gute sensorische Eigenschaften, Verzicht auf ethoxylierte Rohstoffe und Lagerstabilität.

Das Emulsionskonzentrat enthält als Komponente (E) bei 20 °C flüssige Ölkomponente(n) mit einer Polarität von größer 30 - 40 mN/m.

Der Gehalt der Komponente (E), bezogen auf das Emulsionskonzentrat, beträgt in der Regel 3 bis 25 Gew.-%, bevorzugt 5 - 20 Gew.- %., noch bevorzugter 7 - 15 Gew.-%.

In einer bevorzugten Ausführungsform der Erfindung wird als Komponenten (E) eine Verbindung eingesetzt, ausgewählt aus der Gruppe bestehend aus Kohlenwasserstoffen, Dialkylethern, Fettsäureestern mit 12 - 44 C-Atomen, Dialkylcarbonaten, Guerbetalkohole und Siliconöle oder deren Mischungen, sofern sie die erforderliche Polarität von größer 30 bis 40 mN/m aufweist.

Beispiele für geeignete Komponenten (E) sind beispielsweise die folgenden Verbindungen (INCI Bezeichnung gefolgt von Handelsname der Cognis GmbH): Dicaprylyl Ether (Cetiol® OE), Dicarprylyl Carbonate (Cetiol®CC, Propylheptyl Caprylate (Cetiol®Sensoft), Hexyl Laurate (Cetiol®A), sowie die unter den INCI Bezeichnung Coco caprylate sowie Caprylyl caprylate caprate erhältlichen Substanzen.

Das Emulsionskonzentrat enthält als Komponente (F) bei 20 °C flüssige Ölkomponente(n) mit einer Polarität von 5 - 30 mN/m.

Der Gehalt der Komponente (F), bezogen auf das Emulsionskonzentrat, beträgt in der Regel 15 bis 40 Gew.-%, bevorzugt 20 - 35 Gew.- %.

In einer bevorzugten Ausführungsform der Erfindung wird als Komponenten (F) eine Verbindung eingesetzt, ausgewählt aus der Gruppe bestehend aus Triglyceriden, Alkylbenzoaten, Fettsäureestern mit 12 - 44 C-Atomen oder deren Mischungen, sofern sie die erforderliche Polarität von 5 bis 30 bis 40 mN/m aufweist.

Beispiele für geeignete Komponenten (F) sind beispielsweise die folgenden Verbindungen (INCI Bezeichnung gefolgt von Handelsname der Cognis GmbH): Caprylic / Capric Triglyceride (Myrito® 312), Olus Oil (Cegesoft® PS 6), C 12-15 Alkyl Benzoate (Cetiol®AB), Hexyldecanol (and) Hexyldecyl Laurate (Cetiol® PGL), Ethylhexyl Palmitate (Cegesoft® C 24), Mandelöl.

Das Gewichtsverhältnis der Komponenten (E) und (F) zueinander beträgt erfindungsgemäß 1:1 bis 1:3.

Die Ölkomponenten (E) und (F) können Öle, Fette, Wachse sowie beliebige Mischungen daraus enthalten. Als Ölkomponenten (E) und (F) eignen sich alle bei 20 °C flüssigen Fettstoffe oder Fettstoffgemische, d. h. auch Gemische aus flüssigen und darin gelösten, festen Fettstoffen oder Paraffinen, solange diese Gemische bei 20 °C flüssig sind und die erforderliche Polarität aufweisen.

Als "flüssig" werden Komponenten bezeichnet, deren Viskosität (bei 20°C) unter 20 Pas liegt (gemessen mit einem Brookfield Rotationsviskosimeter Typ RVF, Spindel 4, 10 UpM, 23 °C)

Nachfolgend werden Beispiele für Komponenten der lipophilen Phase angegeben. Der Fachmann kann hieraus die für die Erfindung geeigneten Komponenten anhand der Polarität der Komponenten auswählen, indem er die Polarität der Substanzen in Publikationen nachschlägt oder wie oben beschrieben nach der ASTM Methode D971-99a (reapproved 2004) bestimmt.

Unter dem Begriff "Öle" (synonym verwendet: Ölkomponente) werden wasserunlösliche, bei 20 °C flüssige, organische Verbindungen mit relativ niedrigem Dampfdruck bezeichnet. Das gemeinsame Merkmal der Öle ist nicht ihre übereinstimmende chemische Konstitution, sondere ihre ähnliche physikalische Konsistenz.

Als Ölkomponenten sind beispielsweise die nachstehend genannten Verbindungsklassen geeignet, soweit diese bei 20 °C flüssig sind. So z.B. Guerbetalkohole auf Basis von Fettalkoholen mit 6 bis 18, vorzugsweise 8 bis 10 Kohlenstoffatomen (z. B. Eutanol^{®} G) , Ester von linearen C₆-C₂₂-Fettsäuren mit linearen oder verzweigten C₆-C₂₂-Fettalkoholen (z.B. Cetiol^{®} Sensoft) bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat, Erucylerucat und Hexyldecylstearat (Eutanol® G 16 S). Daneben eignen sich Ester von linearen C₆-C₂₂-Fettsäuren mit verzweigten Alkoholen, insbesondere 2- Ethylhexanol, Ester von C₃-C₃₈-Alkylhydroxycarbonsäuren mit linearen oder verzweigten C₆-C₂₂-Fettalkoholen-insbesondere Dioctylmalat -, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane wie z.B. 1 ,3-Dialkylcyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen (Hydagen® HSP, Sovermol® 750, Sovermol® 1102), Siliconöle (Cyclomethicone, Siliciummethicontypen u.a. und/oder aliphatische bzw. naphthenische Kohlenwasserstoffe, wie z.B. wie Mineralöl, Vaseline, Petrolatum, Squalan, Squalen oder Dialkylcyclohexane. Als Öle eignen sich weiterhin Ester von 2-Propylheptanol mit Carbonsäuren, insbesondere mit n-Octansäure (z.B. Cetiol®SenSoft). Als Öle eignen sich weiterhin kurzkettige, lineare Kohlenwasserstoffe, wie beispielsweise n-Undecan, n-Tridecan und n-Pentadecan und Gemische daraus, insbesondere Gemische aus n-Undecan und n-Tridecan.

Als weitere Ölkomponenten kommen beispielsweise Silikonöle in Frage. Sie können als cyclische und/oder lineare Silikonöle vorliegen. Silikonöle sind hochmolekulare synthetische polymere Verbindungen, in denen Silicium- Atome über Sauerstoff-Atome ketten-und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel 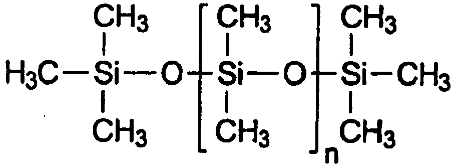 auszeichnen werden auch als Polydimethylsiloxan bzw. Dimethicon (INCI) bezeichnet. Dimethicone gibt es in verschiedenen Kettenlängen bzw. mit verschiedenen Molekulargewichten.

Vorteilhafte Polyorganosiloxane im Sinne der vorliegenden Erfindung sind beispielsweise Dimethylpolysiloxan [Poly (dimethylsiloxan)], welche beispielsweise unter den Handelsbezeichnungen Abil 10 bis 10 000 bei Evonik Goldschmidt erhältlich sind. Ferner vorteilhaft sind Phenylmethylpolysiloxan (INCI: Phenyl Dimethicon, Phenyl Trimethicon), cyclische Silikone (Octamethylcyclotetrasiloxan bzw. Decamethylcyclopentasiloxan), welche nach INCI auch als Cyclomethicon bezeichnet werden, aminomodifizierte Silikone (INCI: Amodimethicone) und Silikonwachse, z. B. Polysiloxan-Polyalkylen-Copolymere (INCI: Stearyl Dimethicon und Cetyl Dimethicon) und Dialkoxydimethylpolysiloxane (Stearoxy Dimethicon und Behenoxy Stearyl Dimethicon), welche als verschiedene Abil-Wax-Typen bei Evonik Goldschmidt erhältlich sind. Aber auch andere Silikonöle sind vorteilhaft im Sinne der vorliegenden Erfindung zu verwenden, beispielsweise Cetyidimethicon, Hexamethylcyclotrisiloxan, Polydimethylsiloxan, Poly (methylphenylsiloxan). Erfindungsgemäß besonders bevorzugte Silikone sind Dimethicon und Cyclomethicon.

Als Ölkomponenten eignen sich auch Polycarbonate, wie beispielsweise in WO 03/041676 beschrieben, auf die hier ausdrücklich Bezug genommen wird.

Als Polycarbonate besonders geeignet ist das unter der INCI Bezeichnung Hydrogenated Dimer Dilinoleyl / Dimethylcarbonate Copolymer, welches als Handelsprodukt Cosmedia^{®} DC von Cognis GmbH erhältlich ist.

Die Dialkylcarbonate und Dialkylether können symmetrisch oder unsymmetrisch, verzweigt oder unverzweigt, gesättigt oder ungesättigt sein und lassen sich nach Reaktionen, die aus dem Stand der Technik hinlänglich bekannt sind, herstellen.

Erfindungsgemäß einsetzbar sind u. a. auch Kohlenwasserstoffe, vorzugsweise mit einer Kettenlänge 8 bis 40 C-Atomen. Sie können verzweigt oder unverzweigt sein, gesättigt oder ungesättigt. Unter diesen sind verzweigte, gesättigte C₈-C₄₀-Alkane bevorzugt. Es können sowohl Reinsubstanzen eingesetzt werden als auch Substanzgemische. Üblicherweise handelt es sich um Substanzgemische verschiedener isomerer Verbindungen. Zusammensetzungen, die Alkane mit 10 bis 30, vorzugsweise 12 bis 20, und besonders bevorzugt 16 bis 20 Kohlenstoffatome aufweisen, sind besonders geeignet, und unter diesen ein Gemisch aus Alkanen, welches wenigstens 10 Gew.-% verzweigte Alkane bezogen auf die Gesamtmenge der Alkane enthält. Vorzugsweise handelt es sich um verzweigte, gesättigte Alkane. Besonders gut geeignet sind Gemische aus Alkanen, welche mehr als 1 Gew.-% 5,8- Diethyldodecan und/oder mehr als 1 Gew.-% Didecen enthalten.

Es kann erfindungsgemäß eine Ölkomponente oder ein Gemisch aus mehreren Ölkomponenten verwendet werden.

### Komponente (A)

Als Komponente (A) werden erfindungsgemäß Alkyl- und/oder Alkenyl(oligo)glykosid(e) mit 6 bis 22 Kohlenstoffatomen im Alk(en)ylrest eingesetzt. Diese Verbindungen sind als Emulgatoren bekannt.

Als Alkyl- und/oder Alkenyloligoglycosid(e) (Komponente (A)) eignen sich insbesondere die Verbindungen der allgemeinen Formel (I):

Gₘ-R¹ (I)

- in der G für einen Zuckerrest mit 5 oder 6 C Atomen steht,
- R' für einen C6 bis C22 Alkyl- und/oder Alkenylrest in Acetalbindung steht,
- m einen mittleren Wert von 1 bis 10, vorzugsweise 1 bis 8, insbesondere 1 bis 3, vorzugsweise 1,2 bis 1,8 darstellt.

Als Alkyl- und/oder Alkenyloligoglycosid(e) (Komponente (A)) eignen sich insbesondere Verbindungen der allgemeinen Formel (I):

Gₘ-R¹ (I)

- in der G für einen Zuckerrest mit 5 oder 6 C Atomen steht,
- R' für einen C6 bis C22 Alkyl- und/oder Alkenylrest in Acetalbindung steht,
- m einen mittteren Wert von 1 bis 3, vorzugsweise 1,2 bis 1,8 darstellt, und wobei mindestens 50 Gew.-% der Alkyl- und/oder Alkenylpolyglycoside einen Rest R' mit einer C Kette größer gleich 12 enthalten.

Ebenfalls erfindungsgemäß bevorzugt ist Komponente (A) ein Alkyloligoglykosidgemisch mit einem Verhältnis von C12-Alkylrest zu C14-Alkylrest von etwa 7 : etwa 3.

Als Alkyl- und/oder Alkenyloligoglycosid(e) (Komponente (A)) eignen sich insbesondere die Verbindungen der allgemeinen Formel (I):

Gₘ-R¹ (I)

- in der G für einen Zuckerrest mit 5 oder 6 C Atomen steht,
- R' für einen C6 bis C22 Alkyl- und/oder Alkenylrest in Acetalbindung steht,
- m einen mittleren Wert von 1 bis 10, vorzugsweise 1 bis 8, insbesondere 1 bis 3, vorzugsweise 1,2 bis 1,8 darstellt
- wobei das Gewichtsverhältnis von Verbindungen der Formel (I), in denen R' einen C12 Alkylrest darstellt zu Verbindungen der Formel (I), in denen R' einen C14 Alkylrest darstellt etwa 7: etwa 3 beträgt

Der Alkyl- bzw. Alkenylrest R' kann sich von primären Alkoholen mit 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Der Alkyl- bzw. Alkenylrest R' kann sich ferner auch von primären Alkoholen mit 12 bis 22, vorzugsweise 12 bis 16 Kohlenstoffatomen ableiten. Typische Beispiele sind Laurylalkohol, Isotridecylalkohol, Myristylalkohol, Cetylalkohol, Isocetylalkohol, Palmoleylalkohol, Stearylalkohol, Isostearylalkohol, Ricinolalkohol, Hydroxystearylalkohol, Dihydroxystearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische.

C₆-C₂₂-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 6 bis 22 C-Atomen. Bezüglich des Glycosidrestes gilt, dass sowohl Monoglycoside, bei denen ein zyklischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Produkte, die unter der Bezeichnung Plantacare^{®} zur Verfügung stehen, enthalten eine glucosidisch gebundene C₈-C₁₆-Alkylgruppe an einem Oligoglucosidrest, dessen mittlerer Oligomerisationsgrad bei 1 bis 2 liegt. Ein bevorzugtes Beispiel ist Laurylglucosid.

Die Emulsionskonzentrate können die Komponente (A) in Mengen von 1 bis 20, vorzugsweise 10 bis 20, insbesondere 12 bis 18 Gew.% - bezogen auf das Gesamtgewicht des Konzentrats - enthalten.

### Komponente (B)

Komponente (B) ist erfindungsgemäß ein Polyglycerolester (gleichbedeutend wird der Begriff Polyglycerinester verwendet). Diese Verbindungen sind als als lipophile Coemulgatoren bekannt.

Polyglycerolester sind Ester von Polyglycerol (gleichbedeutend Polyglycerin) mit beispielsweise Fettsäuren. Als Polylglycerole werden Polymerisationsprodukte von Glycerin eingesetzt, es handelt sich hierbei üblicherweise um Gemische verschiedener Polyglycerole, wie z.B Diglycerol, Triglycerol, Tetraglycerol etc.

In einer bevorzugten Ausführungsform der Erfindung werden Ester von Fettsäuren mit Polyglycerole mit einer durchschnittlichen Länge von 2 bis 12, insbesondere von 2 bis 10, besonders bevorzugt 2 bis 4 Glyceroleinheiten eingesetzt.

Als Fettsäuren, welche mit den Polylgycerolen verestert werden können, eignen sich Verbindungen der allgemeinen Formel (I) R¹-COOH, wobei R' einen linearen oder verzweigten, gesättigten oder ungesättigten, gegebenenfalls Hydroxysubstituierten Rest mit 6 bis 24 C Atomen, vorzugsweise 8bis 20 C Atomen, darstellt.

In einer bevorzugten Ausführungsform der Erfindung wird als Komponente (B) ein Polyglycerolester eingesetzt, der einen HLB-Wert von < 10 aufweist.

### Typische Beispiele für geeignete Polyglycerinester sind

Polyglyceryl-4 Diisostearate/ Polyhydroxystearate/Sebacate (Isolan® GPS), Polyglyceryl-2 Dipolyhydroxystearate (Handelsname Dehymuls® PGPH), Polyglycerin-3-Diisostearate (Handelsname Lameform® TGI), Polyglyceryl-4 Isostearate (Handelsname Isolan® GI 34), Polyglyceryl-3 Oleate (Handelsname z.B. Isolan®GO 33), Diisostearoyl Polyglyceryl-3 Diisostearate (Handelsname Isolan® PDI), Polyglyceryl-3 Beeswax (Handelsname Cera Bellina), Polyglyceryl-4 Caprate (Handelsname Polyglycerol Caprate T2010/90), Polyglyceryl-3 Distearate (Handelsname Cremophor® GS 32), Polyglyceryl Polyricinoleate (Admul® WOL 1403), Polyglyceryl Dimerate Isostearate sowie deren Gemische.

Bevorzugt sind erfindungsgemäß Polyglycerinester von Polyhydroxystearinsäure. Polyglyceryl-2 Dipolyhydroxystearate ist eine erfindungsgemäß bevorzugte Komponente (B). Diese Verbindung ist beispielsweise unter dem Handelsnamen Dehymuls®PGPH von der Fa. Cognis erhältlich oder unter der Bezeichnung AEC Polyglyceryl-2 Dipolyhydroxystearate von der Fa. A & E Connock.

Die Emulsionskonzentrate können die Komponente (B) in Mengen von 1 bis 20, vorzugsweise 5 bis 20, insbesondere 8 bis 18 Gew.% - bezogen auf das Gesamtgewicht des Konzentrats - enthalten.

Erfindungsgemäß ist das Verhältnis der Komponenten (A) zu (B) 1 : (0,6 - 0,8). Die Emulsionskonzentrate können die Komponenten (A) und (B) insgesamt z. B. in Mengen von 2 bis 40, vorzugsweise 10 bis 40, insbesondere 15 bis 36 Gew.-% und besonders bevorzugt 20 bis 35 Gew.-%, bezogen auf das Gesamtgewicht des Konzentrats, enthalten.

### Komponente (C) - Polyole

Als Polyole werden mehrwertige Alkohole, d. h. organische Verbindungen, die mindestens 2 alkoholische Hydroxylgruppen im Molekül tragen, bezeichnet. In einer Ausführungsform der Erfindung enthalten die Polyole 2 bis 6 Hydroxylgruppen pro Molekül. In einer Ausführungsform der Erfindung werden als Polyole niedermolekulare mehrwertige Alkohole eingesetzt, d.h. Verbindungen die 2 bis 18, insbesondere 2 bis 10, vorzugsweise 2 bis 6 C-Atome enthalten.

Die erfindungsgemäßen Polyolen sind dabei von den Komponenten (A), (B) und (D), (E) und (F) verschiedene Stoffe. D.h. falls das erfindungsgemäße Emulsionskonzentrat eine Alkyl - und/oder Alkenyloligoglykosid (A) oder ein Polyglycerolester (B) oder ein Glycerid (D) oder eine Ölkomponenten (E) oder (F) enthält, welche mindestens 2 alkoholische Hydroxylgruppen im Molekül trägt, werden diese zu den entsprechenden Komponente (A), (B), (D), (E) oder (F) gerechnet und nicht zur Komponente (C).

In einer bevorzugten Ausführungsform der Erfindung werden als Polyole (C) Verbindungen eingesetzt, die mindestens 2 Hydroxylgruppen pro Molekül tragen und aus 2 bis 18, vorzugsweise 2 bis 10, insbesondere aus 2 bis 6 C-Atomen bestehen.

In einer bevorzugten Ausführungsform der Erfindung werden als Polyole (C) Verbindungen eingesetzt, die 2 bis 6 Hydroxylgruppen pro Molekül tragen. Besonders bevorzugt sind Polyole (C), die 2 bis 6 Hydroxylgruppen pro Molekül tragen und aus 2 bis 6 C-Atomen bestehen.

Als Polyole (C) können sowohl einzelne Polyole als auch Mischungen aus beliebigen Polyolen eingesetzt werden. In einer bevorzugten Ausführungsform werden als Polyole Mischungen aus mindestens 2, insbesondere mindestens 3 Polyolen eingesetzt.

Die Polyole (C) können noch weitere funktionelle Gruppen, insbesondere Aminogruppen, enthalten bzw. mit Stickstoff modifiziert sein. In einer bevorzugten Ausführungsform enthalten die Polyole außer den Hydroxylgruppen keine weiteren funktionellen Gruppen.

In einer Ausführungsform der Erfindung sind die Polyole (C) ausgewählt aus der Gruppe bestehend aus Glycerin, Diglycerin, Triglycerin, Tetraglycerin, Alkylenglykolen und Polyalkylenglykolen.

Als Alkylenglykole geeignet sind Ethylenglycol (=1,2 Ethandiol), Diethylenglycol (=2,2'-Oxydiethanol; HO-(CH₂)₂-O-(CH₂)₂-OH), Triethylenglycol (=2,2'-(Ethylendioxy)diethanol), 1,2-Propylenglycol, 1,3-Propylenglycol, Butylenglycole (=Butandiole) wie 1,2-Butylenglycol, 1,3 Butylenglycol, 1,4 Butyleneglycol, 2,3 Butyleneglycol; Pentan-1,5-diol; Pentan-1,2-diol; meso-Pentan-2,4-diol, (2R, 4R)-Pentan-2,4-diol; (2S, 4S)-Pentan-2,4-diol, Hexandiole, wie beispielsweise Hexylenglycol (= 2-Methylpentan-2,4-diol), Heptandiole, Octandiole und Decandiole.

Als Polyalkylenglycole werden überwiegend lineare, zum Teil aber auch verzweigte Polyether bezeichnet, die aus der Polykondensation von Glycolen entstehen. Die technisch wichtigen Vertreter dieser Polyether-Polyole sind die Polyethylenglycole, Polypropylenglycole, Polyethylene/Polypropyleneglycole sowie Polytetramethylenglycole bzw. deren analoge Verbindungen, die durch ringöffnende Polymerisation von Ethylenoxid, Propylenoxid bzw. Tetrahydrofuran hergestellt werden.

Besonders bevorzugt im Sinne der Erfindung sind als Polyalkylenglycole Polyethyleneglycole und/oder Polypropyleneglycole und/oder Polyethylen-Polypropyleneglycole mit einem durchschnittlichen Molekulargewicht von 100 bis 1000 Dalton.

Die bevorzugten Polyalkyleneglycole folgend dabei der allgemeinen Formel

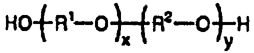

Sind R¹ und R² = (CH₂)₂ bezeichnet die Formel Polyethyleneglykole, sind R¹ und R² = CH₂-CH(CH₃) bezeichnet die Formel Polyproplyeneglykole. Ist R¹ = (CH₂)₂ und R² = CH₂-CH(CH₃) bezeichnet die Formel Polyethylene/Polypropyleneglykole.

Diese Verbindungen sind kommerziell beispielsweise unter der INCI Bezeichnung (in Klammer Handelsname und Hersteller) erhältlich:
INCI: PEG-4 (Polyglykol 200 USP, Clariant International; Pluracare E 200, BASF Corp.)
INCI: PEG-12 (Polyglykol 600, Clariant International; Pluracare E 600, BASF Corp.)
INCI: PPG-3 (Newpol PP-200, Sanyo Chemical Industries)

In einer weiteren Ausführungsform der Erfindung sind die Polyole (c) ausgewählt aus der Gruppe die gebildet wird von
- Glycerin, Diglycerin, Triglycerin, Tetraglycerin
- Alkylenglycolen ausgewählt aus der Gruppe bestehend aus Ethylenglycol (=1,2 Ethandiol), Diethylenglycol (=2,2'-Oxydiethanol; HO-(CH₂)₂-O-(CH₂)₂-OH), Triethylenglycol (=2,2'-(Ethylendioxy)diethanol), 1,2-Propylenglycol, 1,3-Propylenglycol, Butylenglycole (=Butandiole) wie 1,2-Butylenglycol, 1,3 Butylenglycol, 1,4 Butyleneglycol, 2,3 Butyleneglycol; Pentan-1,5-diol; Pentan-1,2-diol; meso-Pentan-2,4-diol, (2R, 4R)-Pentan-2,4-diol; (2S, 4S)-Pentan-2,4-diol, Hexandiole, wie beispielsweise Hexylenglycol (= 2-Methylpentan-2,4-diol), Heptandiole, Octandiole und Decandiole.
- Polyalkyleneglycolen mit einem durchschnittlichen Molekulargewicht von 100 bis 1000 Dalton
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- kurzkettige Alkyglucoside, insbesondere solche mit 1 bis 5 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

In einer bevorzugten Ausführungsform der Erfindung wird als Polyol (C) mindestens eine Verbindung eingesetzt, ausgewählt aus der Gruppe, bestehend aus Glycerin, 1,2-Propylenglykol, Sorbit, Butylenglykolen und Hexylenglykol.

Die Emulsionskonzentrate können die Komponente (C) in Mengen von 1 bis 20, vorzugsweise 10 bis 20, insbesondere 12 bis 18 Gew.% - bezogen auf das Gesamtgewicht des Konzentrats - enthalten. Erfindungsgemäß beträgt das Gewichtsverhältnis der Komponenten (A), (B) und (C) 1 : (0,6 - 0,8) : (0,9 - 1,1).

### Komponente (D) - Glyceride

Als Komponente (D) werden erfindungsgemäß Glyceride eingesetzt, die einen Schmelzpunkt von größer gleich 20 °C aufweisen, insbesondere solche Glyceride, die einen Schmelzpunkt von größer gleich 30 °C aufweisen. Diese haben ebenfalls Coemulgatorwirkung. Unter der Bezeichnung Glyceride fasst man Ester von Carbonsäuren, insbesondere von Fettsäuren mit Glycerin zusammen. Ja nach Anzahl der Säurereste unterscheidet man zwischen Monoacylglycerolen (= Monoglyceriden), Diacylglycerolen (= Diglyceriden) und Triacylglycerolen (=Triglyceriden). Mit Glyceriden sind erfindungsgemäß Monoglyceride, Diglyceride sowie Triglyceride und beliebige Mischungen dieser Verbindungen umfasst. Monoglyceride sowie Diglyceride werden auch als Partialglyceride oder Glycerinpartialester bezeichnet.

Typische Beispiele für geeignete Partialglyceride sind Mono- und/oder Diglyceride von C12 bis C22 Fettsäuren mit Glycerin sowie deren technische Gemische. Beispielsweise seien genannt langkettige Hydroxyfettsäuremonoglyceride, langkettige Hydroxyfettsäurediglyceride, Isostearinsäuremonoglycerid, Isostearinsäurediglycerid, Ölsäuremonoglycerid , Ölsäurediglycerid, Ricinolsäuremonoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid.

Typische Beispiele für geeignete Partialglyceride sind Mono- und/oder Diglyceride von Dicarbonsäuren mit 4 bis 8 C-Atomen mit Glycerin sowie deren technische Gemische. Besonders geeignet sind solche Partialglyceride, die einen Schmelzpunkt von >30°C aufweisen. Beispielsweise seien genannt Weinsäuremonoglycerid, Weinsäurediglycerid, Citronensäuremonoglycerid, Citronensäurediglycerid, Äpfelsäuremonoglycerid, Äpfelsäurediglycerid sowie deren technische Gemische, die untergeordnet aus dem Herstellungsprozess noch geringe Mengen an Triglycerid enthalten können.

Als Komponente (D) sind erfindungsgemäß insbesondere Mono- und Diglyceride bzw. Mischungen dieser Partialglyceride einsetzbar. Zu den erfindungsgemäß einsetzbaren Glyceridgemischen zählen die von der Cognis GmbH vermarkteten Produkte Novata® AB und Novata® B (Gemisch aus C12-C18- Mono-, Di- und Triglyceriden) sowie Cutina® MD oder Cutina® GMS (Glycerylstearat).

In einer bevorzugten Ausführungsform der Erfindung werden als Komponenten (D) Glyceride von C18 Fettsäuren eingesetzt. Vorzugsweise beträgt der Anteil an Glycerinmonoestern der C18 Fettsäuren - bezogen auf das Gesamtgewicht aller Glyceride von C18 Fettsäurengrößer gleich 90 Gew.%, vorzugsweise größer gleich 95 Gew.-%. Als C18 Fettsäuren sind insbesondere einfach, zweifach oder dreifach ungesättigte C 18 Fettsäuren geeignet. In einer bevorzugten Ausführungsform der Erfindung werden als Komponente (D) Glyceride von C18 Fettsäuren eingesetzt, wobei der Anteil der Glyceride mit ungesättigten C18 Fettsäuren (bezogen auf das Gesamtgewicht aller Glyceride von C18 Fettsäuren) größer gleich 80 Gew.-%, vorzugsweise größer gleich 90 Gew.-% beträgt.

Die Komponente (D) kann erfindungsgemäß z. B. in einem Anteil von etwa 1 bis 10 Gew.-%, bevorzugt etwa 2 bis 8 Gew.-%, bezogen auf das Emulsionskonzentrat, enthalten sein. Erfindungsgemäß beträgt das Gewichtsverhältnis der Komponente (A) zur Komponente (D) 4 : 1 bis 1,5 : 1 , insbesondere 3,5 : 1 bis 2:1.

### Wässriger Anteil

Die erfindungsgemäßen Konzentrate weisen einen Wassergehalt auf von kleiner gleich 20 Gew.-%, vorzugsweise kleiner gleich 18 Gew.-% auf.

### Ethoxylierte Substanzen

In einer bevorzugten Ausführungsform der Erfindung enthalten die Emulsionskonzentrate einen Anteil an ethoxylierten Substanzen der unter 10 Gew.-%, insbesondere unter 5 Gew.-%, vorzugsweise unter 2 Gew.-%, insbesondere unter 1 Gew.-% bezogen auf das Emulsionskonzentrat, beträgt. In einer besonders bevorzugten Ausführungsform der Erfindung sind die Emulsionskonzentrate frei von ethoxylierten Substanzen.

### Herstellung der erfindungsgemäßen Emulsionskonzentrate

Eine Möglichkeit der Herstellung der erfindungsgemäßen Emulsionskonzentraten ist wie folgt: Alle Komponenten (A) bis (F) werden zusammen mit dem Wasser unter Rühren auf 70 bis 75 °C für 60 min. erhitzt. Anschließend wir das Konzentrat auf 30 °C abgekühlt und gegebenenfalls wird der pH-Wert auf 5,0 bis 6,0 eingestellt.

### Verwendung der Emulsionskonzentrate

Die erfindungemäßen Emulsionskonzentrate können direkt als kosmetische oder pharmazeutische Zubereitung verwendet werden. Ein weiterer Gegenstand der Erfindung betrifft daher die Verwendung der erfindungsgemäßen Emulsionskonzentrate als kosmetische oder pharmazeutische Zubereitungen.

Die erfindungsgemäßen Emulsionskonzentrate sind feinteilig und sehr lagerstabil, sie eignen sich daher ganz besonders als vorgefertigter Emulsionsbaustein, der aufgrund seiner Fliessfähigkeit gut für die Lagerung und den Transport an einen Verarbeitungsort mit geringer technischer Ausrüstung geeignet ist, um dort mit einfachsten Mitteln brauchbare kosmetische und pharmazeutische Emulsionen, insbesondere Öl-in-Wasser Emulsionen herzustellen.

Ein Gegenstand der Erfindung betrifft demnach die Verwendung der erfindungsgemäßen Emulsionskonzentrate zur Herstellung von kosmetischen oder pharmazeutischen Zubereitungen.

Das erfindungsgemäße Emulsionskonzentrat eignet sich insbesondere zur Herstellung kosmetischer oder pharmazeutischer Öl-in-Wasser-Emulsionen. Dabei kann beispielsweise das Emulsionskonzentrat vorgelegt werden und die wässrige Phase zur Vorlage gegeben werden, ebenso kann die wässrige Phase vorgelegt werden und das Emulsionskonzentrat zu der wässrigen Vorlage gegeben werden. Möglich ist auch das Emulsionskonzentrat mit weiteren lipophilen Komponenten zu mischen dieses zur Vorlage (wässrige Phase) zu geben oder das gegebenenfalls mit weiteren lipophilen Komponenten gemischte Emulsionskonzentrat vorzulegen und die wässrige Phase zu dieser Vorlage zu geben.

Die Herstellung der kosmetischen oder pharmazeutischen Zubereitungen kann dabei ohne weitere Wärmezufuhr erfolgen.

Die liphophile Phase, mit der das Emulsionskonzentrat verdünnt wird, kann beliebige lipophile Bestandteile, z.B. liphophile kosmetische Wirkstoffe, enthalten. Als lipophile Phase eignen sich beispielsweise alle als Komponente (E) und (F) geeigneten Verbindungen.

In der Regel werden 1 bis 50, bevorzugt 2 bis 30, insbesondere 4 bis 10 Gew.-% des Emulsionskonzentrates für die Herstellung der kosmetischen oder pharmazeutischen Zubereitungen eingesetzt.

### Kosmetische oder pharmazeutische Zubereitungen

Ein weiterer Gegenstand der Erfindung betrifft kosmetische oder pharmazeutische Zubereitungen, enthaltend folgende Komponenten:
(A) Alkyl- und/oder Alkenyloligoglykosid(e) mit 6 bis 22 Kohlenstoffatomen im Alk(en)ylrest,
(B) Polyglycerolester,
(C) Polyol(e),
(D) Glycerid(e), die einen Schmelzpunkt größer gleich 20 °C aufweisen
(E) bei 20 °C flüssige Ölkomponente(n) mit einer Polarität von größer 30 - 40 mN/m und
(F) bei 20 °C flüssige Ölkomponente(n) mit einer Polarität von 5 - 30 mN/m,
wobei
- die Komponenten (A), (B) und (C) ein Gewichtsverhältnis A : B : C von 1 : (0,6 - 0,8) : (0,9 - 1,1) aufweisen,
- die Komponenten (A) und (D) ein Gewichtsverhältnis A : D von 4 : 1 bis 1,5 : 1 aufweisen,
- die Komponenten (E) und (F) ein Gewichtsverhältnis E : F von 1 : 1 bis 1 : 3 aufweisen, und

Überraschenderweise wurde gefunden, dass durch die Auswahl der Komponenten sowie die erfindungsgemäßen Verhältnisse der Komponenten zueinander stabile, feinteilige Zubereitungen, insbesondere Öl-in-Wasser Emulsionen erhalten werden können, die ohne ethoxylierte Substanzen formuliert werden können.

In einer bevorzugten Ausführungsform der Erfindung handelt es sich bei den erfindungsgemäßen Zubereitungen um Öl-in-Wasser Emulsionen.

Die erfindungsgemäßen Zubereitungen können beispielsweise hergestellt werden, indem man die erfindungsgemäßen Emulsionskonzentrate entsprechend verdünnt. Im einfachsten Fall erfolgt die Verdünnung mit Wasser, da so die erfindungsgemäßen Verhältnisse der Komponenten nicht verändert werden.

Die kosmetischen oder pharmazeutischen Zubereitungen weisen üblicherweise eine mittlere Teilchengröße von unter 1 µm, insbesondere von unter 500 nm, vorzugsweise von unter 250 nm, besonders bevorzugt von unter 200 nm auf.

### Weitere Bestandteile

Neben den genannten Bestandteilen können in den Emulsionskonzentraten bzw. in den erfindungsgemäßen kosmetischen und/oder pharmazeutischen Zubereitungen weitere, von den Komponenten (A), (B), (C), (D), (E) und (F) verschiedene, übliche Bestandteile in üblichen Anteilen enthalten sein. Hierzu gehören z.B. Tenside, Konservierungsmittel, biogene Wirkstoffe, Verdickungsmittel, Überfettungsmittel, Stabilisatoren, Polymere, Antioxidantien, Filmbildner, Quellmittel, Insektenrepellentien, Hydrotrope, Solubilisatoren, Parfümöle, Farbstoffe, Pigmente, UV-Filter etc.

Sollten die weiteren Bestandteile wasserunlöslich sein, sind sie der lipophilen Phase zuzurechnen. Je nach Polarität sind sie dann gegebenenfalls der Komponente (E) oder (F) zuzuordnen.

Die erfindungsgemäßen Emulsionskonzentrate enthalten die weiteren Bestandteile üblicherweise in Mengen von insgesamt kleiner gleich 25 Gew.%, insbesondere kleiner gleich 20 Gew.%, besonders bevorzugt unter 10 Gew.-% bezogen auf das Gesamtgewicht des Emulsionskonzentrates.

Die erfindungsgemäßen kosmetischen und/oder pharmazeutischen Zubereitungen enthalten die weiteren Bestandteile üblicherweise in Mengen von insgesamt kleiner gleich 25 Gew.%, insbesondere kleiner gleich 20 Gew.%, besonders bevorzugt unter 10 Gew.-% bezogen auf das Gesamtgewicht der Zubereitung

### Tenside

Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekularteil, für eine Herabsetzung der Oberflächenspannung des Wassers, die Benetzung der Haut, die Erleichterung der Schmutzentfernung und -lösung, ein leichtes Abspülen und - je nach Wunsch- für Schaumregulierung.

Als Tenside werden üblicherweise oberflächenaktive Substanzen verstanden, die einen HLB-Wert von größer 20 aufweisen. Als oberflächenaktive Stoffe können anionische, nichtionische, kationische und/oder amphotere bzw. zwitterionische Tenside enthalten sein. In tensidhaltigen kosmetischen Zubereitungen ist vorzugsweise wenigstens ein anionisches Tensid enthalten.

Die erfindungsgemäßen Konzentrate bzw. Zubereitungen können die Tenside üblicherweise in einer Menge von 0 bis 40 Gew.-%., vorzugsweise 0,05 bis 30 Gew.-% insbesondere 0,05 bis 20 Gew.-%, vorzugsweise 0,1 bis 15 Gew.-% und insbesondere 0,1 bis 10 Gew.-%, bezogen auf das Gesamtgewicht der Konzentrate bzw. Zubereitungen, enthalten.

Typische Beispiele für nichtionische Tenside sind Fettalkoholpolyglycolether, Alkylphenolpolyglycolether, Fettsäurepolyglycolester, Fettsäureamidpolyglycolether, Fettamin-polyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾-Gruppe tragen. Besonders geeignete zwitterionische Tenside sind die so genannten Betaine wie die N-Alkyl-N,N-dimethylammoniumglycinate, beispielsweise das Kokosalkyldimethylammoniumglycinat, N-Acyl-aminopropyl-N,N-dimethylammonium-glycinate, beispielsweise das Kokosacylaminopropyldimethylammoniumglycinat, und 2-Alkyl-3-carboxylmethyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls, insbesondere als Co-Tenside geeignet, sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N- Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂-₁₈-Acylsarcosin.

Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside und/ oder deren Gemische mit Alkyloligoglucosidcarboxylaten, Fettsäureglucamide, Alkylamidobetaine, Amphoacetale und/oder Proteinfettsäurekondensate, letztere vorzugsweise auf Basis von Weizenproteinen bzw. deren Salzen.

Anionische Tenside sind gekennzeichnet durch eine wasserlöslich machende, anionische Gruppe wie z. B. eine Carboxylat-, Sulfat-, Sulfonat- oder Phosphat-Gruppe und einen lipophilen Rest. Hautverträgliche anionische Tenside sind dem Fachmann in großer Zahl aus einschlägigen Handbüchern bekannt und im Handel erhältlich. Es handelt sich dabei insbesondere um Alkylsulfate in Form ihrer Alkali-, Ammonium- oder Alkanolammoniumsalze, Alkylethersulfate, Alkylethercarboxylate, Acylisethionate, Acylsarkosinate, Acyltaurine mit linearen Alkyl- oder Acylgruppen mit 12 bis 18 C-Atomen sowie Sulfosuccinate und Acylglutamate in Form ihrer Alkali- oder Ammoniumsalze.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymisch-ethersulfate, Monoglycerid-(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Als kationische Tenside sind insbesondere quartäre Ammoniumverbindungen verwendbar. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie beispielsweise die unter dem Warenzeichen Stepantex^{®} vertriebenen Dialkylammoniummethosulfate und Methylhydroxyalkyldialkoyloxyalkylammoniummethosulfate und die entsprechenden Produkte der Dehyquart^{®}-Reihe, als kationische Tenside eingesetzt werden. Unter der Bezeichnung "Esterquats" werden im allgemeinen quaternierte Fettsäuretriethanolaminestersalze verstanden. Sie können den erfindungsgemäßen Zubereitungen einen besonderen Weichgriff verleihen. Es handelt sich dabei um bekannte Stoffe, die man nach den einschlägigen Methoden der organischen Chemie herstellt. Weitere erfindungsgemäß verwendbare kationische Tenside stellen die quaternisierten Proteinhydrolysate dar.

Als **Konservierungsmittel** eignen sich beispielsweise Phenoxyethanol, Ethylhexylglycerin, Dicaprylyl Glycol, Formaldehydlösung, Parabene, Pentandiol, Mischungen aus Phenoxyethanol und Ethylhexylglycerin (wie sie beispielsweise unter dem Handelsnamen Euxyl PE 9010 erhältlich sind), oder Sorbinsäure sowie die unter der Bezeichnung Surfacine® bekannten Silberkomplexe und die in Anlage 6, Teil A und B der Kosmetikverordnung aufgeführten weiteren Stoffklassen.

In einer bevorzugten Ausführungsform der Erfindung wird das Konservierungsmittel ausgewählt aus der Gruppe bestehend aus Phenoxyethanol, Formaldehydlösung, Parabenen, organische Säuren und Mischungen daraus, gegebenenfalls in Kombination mit Pentandiol und/oder Ethylhexylglycerin bzw. Octandiol und Ethylhexylglycerin (Handelsname Sensiva SC 10).

In einer bevorzugten Ausführungsform der Erfindung enthalten die erfindungsgemäßen Konzentrate bzw. Zubereitungen als biogenen Wirkstoff mindestens eine Verbindung ausgewählt aus Vitaminen, Allantoin, Bisabolol und Pflanzenextrakten.

In einer bevorzugten Ausführungsform der Erfindung enthalten die erfindungsgemäßen Konzentrate bzw. Zubereitungen als biogenen Wirkstoff mindestens eine Verbindung ausgewählt aus Tocopherol, Tocopherolacetat, Tocopherolpalmitat, Ascorbinsäure, (Desoxy)Ribonucleinsäuren und deren Fragmentierungsprodukten, β-Glucanen, Retinol, Bisabolol, Allantoin, Phytantriol, Panthenol, AHA-Säuren, Aminosäuren, Ceramiden, Pseudoceramiden, essentiellen Ölen, Pflanzenextrakten, wie z. B. Aloe vera, Prunusextrakt, Bambaranussextrakt und Vitaminkomplexe und Mischungen daraus.

In einer Ausführungsform der Erfindung enthalten die erfindungsgemäßen Konzentrate bzw. Zubereitungen als weiteren Bestandteil mindestens ein Verdickungsmittel.

Als Verdickungsmittel eignen sich beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, Polyvinylalkohol, Polyvinylpyrrolidon und Bentonite wie z. B. Bentone® Gel VS-5PC (Rheox).

Als Insekten-Repellentien kommen beispielsweise N,N-Diethyl-m-toluamid, 1 ,2-Pentandiol oder 3-(N-n-Butyl-N-acetyl-amino)-propionsäureethylester), welches unter der Bezeichnung Insect Repellent® 3535 von der Merck KGaA vertrieben wird, sowie Butylacetylaminopropionate in Frage.

Als Parfümöle seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

Der Begriff Pigment umfasst Partikel jeder Form, die weiß oder gefärbt, organisch oder anorganisch sind, in den Zubereitungen nicht löslich sind, und dem Zweck dienen, die Zubereitung zu färben. In einer bevorzugten Ausführungsform werden anorganische Pigmente verwendet, besonders bevorzugt sind Metalloxide.

Als **anorganische Pigmente** seien exemplarisch genannt: Titandioxid, optional oberflächenbeschichtet, Zirkonium oder Ceriumoxide und Zink-, Eisen- (Schwarz, Gelb oder Rot) und Chromoxide, Manganviolett, Ultramarine Blau, Chromhydrate und Eisen(III)blau, Metallpulver wie Aluminiumpulver oder Kupferpulver.

In einer bevorzugten Ausführungsform der Erfindung ist das Pigment ausgewählt aus den anorganischen Pigmenten, vorzugsweise aus den Metalloxiden. In einer bevorzugten Ausführungsform ist das Pigment ausgewählt aus der Gruppe bestehend aus Titandioxid, Zinkoxid, Eisenoxid und Mischungen daraus. Die Pigmente können sowohl einzeln als auch in Mischungen vorliegen. Bevorzugt im Sinne der vorliegenden Erfindung sind Pigmentmischungen aus Weiß-Pigmenten (z. B. Kaolin, Titandioxid oder Zinkoxid) und anorganischen Farbpigmenten (z. B. Eisenoxid Pigmente, Chromoxide), wobei die Pigmente beschichtet ("gecoatet") oder unbeschichtet vorliegen können. Unter den Farbpigmenten sind Eisenoxide besonders bevorzugt.

Vorteilhaft im Sinne der vorliegenden Erfindung können das oder die Pigmente auch aus der Gruppe der Effektpigmente gewählt werden, welche der kosmetischen Zubereitung neben der reinen Farbe eine zusätzliche Eigenschaft - wie z. B. eine Winkelabhängigkeit der Farbe (changieren, Flop), Glanz (nicht Oberflächenglanz) oder Textur - verleihen. Solche Effektpigmente werden erfindungsgemäß vorteilhaft zusätzlich zu einem oder mehreren Weiß- und/oder Farbpigmenten eingesetzt.

Die bedeutendste Gruppe der Effektpigmente stellen die Glanzpigmente dar, zu denen nach DIN 55944: 2003-11 die Metalleffektpigmente und die Perlglanzpigmente gehören. Einige spezielle Effektpigmente lassen sich diesen beiden Gruppen nicht zuordnen, z. B. plättchenfoermiges Graphit, plättchenförmiges Eisenoxid und mikronisiertes Titandioxid, wobei mikronisiertes Titandioxid keinen Glanzeffekt, sondern einen winkelabhängigen Lichtstreueffekt erzeugt. Bei den Glanzpigmenten nach DIN 55943: 2001-10 handelt es sich vorwiegend um plättchenfoermige Effektpigmente. Parallel orientiert zeigen Glanzpigmente einen charakteristischen Glanz. Die optische Wirkung von Glanzpigmenten beruht auf der gerichteten Reflexion an metallischen Teilchen (Metalleffektpigmente), an transparenten Teilchen mit hoher Brechzahl (Perlglanzpigmente) oder auf dem Phaenomen der Interferenz (Interferenzpigmente) (DIN 55944: 2003-11).

Beispiele für erfindungsgemäß bevorzugte handelsübliche Effektpigmente sind: Timiron and #174; von Merck, Iriodin and #174; von Merck (Perl- und Farbglanzpigmente für dekorative technische Anwendungen), Xirallic and #174; von Merck (farbintensive Kristalleffektpigmente).

Ferner können die erfindungsgemäßen Konzentrate bzw. Zubereitungen vorteilhaft auch organische Farbpigmente enthalten, d. h. organische Farbstoffe, welche in der Zubereitung praktisch unlöslich sind. Nach DIN 55944: 1990-04 können organische Pigmente nach chemischen Gesichtspunkten in Azopigmente und polycyclische Pigmente sowie nach farblichen Gesichtspunkten in Bunt- oder Schwarzpigmente eingeteilt werden. Organische Weißpigmente sind ohne praktische Bedeutung. Die Pigmente können vorteilhaft im Sinne der vorliegenden Erfindung auch in Form kommerziell erhältlicher öliger oder wässriger Vordispersionen zur Anwendung kommen.

Es ist ferner möglich, dass die erfindungsgemäßen Konzentrate bzw. Zubereitungen einen oder mehrere **Farbstoffe** enthalten.

Die Farbstoffe können sowohl synthetischen als auch natürlichen Ursprungs sein. Eine Liste von geeigneten Farbstoffen findet sich in EP 1 371 359 A2**,** S.8, Z. 25-57, S.9 und S.10 sowie S.11, Z. 1 bis 54, auf welche hiermit explizit Bezug genommen wird.

Als Farbstoffe und Pigmente eignen sich insbesondere die gemäß Annex IV der Kommissions Direktive zugelassenen Farbstoffe und Pigmente (in der Fassung: Commission Directive 2007/22/EC of 17 April 2007 amending Council Directive 76/768/EEC, concerning cosmetic products, for the purposes of adapting Annexes IV and VI thereto to technical progress), auf die hiermit explizit Bezug genommen wird.

Erfindungsgemäß sind als **UV-Lichtschutzfilter** bei Raumtemperatur flüssige oder kristalline organische Substanzen (Lichtschutzfilter) geeignet, die in der Lage sind, ultraviolette Strahlen zu absorbieren und die aufgenommene Energie in Form längerwelliger Strahlung, z.B. Wärme wieder abzugeben. UV-Filter können öllöslich oder wasserlöslich sein. Als typische öllösliche UV-B-Filter bzw. Breitspektrum-UV A/B-Filter sind z.B. zu nennen:
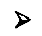 3-Benzylidencampher bzw. 3-Benzylidennorcampher (Mexoryl SDS 20) und dessen Derivate, z.B. 3-(4-Methylbenzyliden)campher wie in der EP 0693471 B1 beschrieben
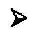 3-(4'-Trimethylammonium) benzyliden- bornan-2-on-methylsulfat (Mexoryl SO)
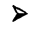 3,3'-(1,4-Phenylendimethin)-bis (7,7- dimethyl-2-oxobicyclo-[2.2.1] heptan-1-methansulfonsäure) and salts (Mexoryl SX)
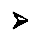 3-(4'-Sulfo)-benzyliden-bornan-2-on and salts (Mexoryl SL)
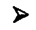 Polymer von N-[2(und 4)- (2-oxoborn-3-ylidenmethyl)benzyl]acrylamid Mexoryl SW)
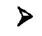 2-(2H-Benzotriazol-2-yl)-4-methyl-6-(2-methyl-3-(1,3,3,3-tetramethyl-1-(trimethylsilyloxy)disiloxanyl)propyl) phenol (Mexoryl XL)
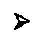 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
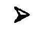 Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene);
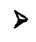 Ester der Salicylsäure, vorzugsweise Salicylsäure-2-ethylhexylester, Salicylsäure-4-isopropylbenzylester, Salicylsäurehomomenthylester;
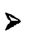 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
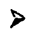 Ester der Benzalmalonsäure, vorzugsweise 4-Methoxybenzmalonsäuredi-2-ethylhexylester;
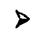 Triazinderivate, wie z.B. 2,4,6-Trianilino-(p-carbo-2'-ethyl-1'-hexyloxy)-1,3,5-triazin und 2,4,6-Tris[p-(2-ethylhexyl-oxycar-bonyl) anilino]-1,3,5-triazin (Uvinul T 150) wie in der EP 0818450 A1 beschrieben oder 4,4'-[(6-[4-((1,1-Dimethylethyl)amino-carbonyl) phenyl-amino]-1,3,5-triazin-2,4- diyl)diimino] bis(benzoesäure-2- ethylhexylester) (Uvasorb® HEB);
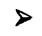 2,2(-Methylen-bis(6-(2H-benzotriazol-2-yl)-4- (1,1,3,3-tetramethyl-butyl)phenol) (Tinosorb M);
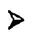 2,4-Bis[4-(2-ethylhexyloxy)-2- hydroxyphenyl]-6-(4- methoxyphenyl)-1,3,5- triazin (Tinosorb S);
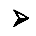 Propan-1,3-dione, wie z.B. 1-(4-tert.Butylphenyl)-3-(4'methoxyphenyl)propan-1,3-dion;
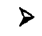 Ketotricyclo(5.2.1.0)decan-Derivate, wie in der EP 0694521 B1 beschrieben;
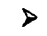 Dimethicodiethylbenzalmalonate (Parsol SLX).

Als wasserlösliche UV - Filter kommen in Frage:
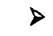 2-Phenylbenzimidazol-5-sulfonsäure und deren Alkali-, Erdalkali-, Ammonium-, Alkylammonium-, Alkanolammonium- und Glucammoniumsalze;
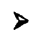 2,2(-(1,4-Phenylen)bis(1H-benzimidazol- 4,6-disulfon-säure, Mononatriumsalz) (Neo Heliopan AP)
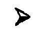 Sulfonsäurederivate von Benzophenonen, vorzugsweise 2-Hydroxy-4-methoxybenzophenon-5-sulfonsäure und ihre Salze;
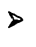 Sulfonsäurederivate des 3-Benzylidencamphers, wie z.B. 4-(2-Oxo-3-bomylidenmethyl)-benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der DE 19712033 A1 (BASF) sowie Benzoic Acid, 2-[4-(Diethylamino)-2-Hydroxybenzoyl]-, Hexyl Ester (Uvinul® A plus).

Die UV-A und UV-B-Filter können selbstverständlich auch in Mischungen eingesetzt werden. Besonders günstige Kombinationen bestehen aus den Derivaten des Benzoylmethans, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Als UV-Lichtschutzfilter eignen sich insbesondere die gemäß Annex VII der Kommissions Direktive (in der Fassung Commission Directive 2005/9/EC of 28 January 2005 amending Council Directive 76/768/EEC, concerning cosmetic products, for the purposes of adapting Annexes VII thereof to technical progress) zugelassen Stoffe, auf die hier explizit Bezug genommen wird.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und auch für die dekorative Kosmetik verwendet. Die Partikel sollten einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex0 T, Eusolex® T-2000, Eusolex® T-Aqua, Eusolex® AVO, Eusolex® T-ECO, Eusolex® T-OLEO und Eusolex® T-S (Merck). Typische Beispiele für sind Zinkoxide, wie z.B. Zinc Oxide neutral, Zinc Oxide NDM (Symrise) oder Z-Cote® (BASF) oder SUNZnO-AS und SUNZnO-NAS (Sunjun Chemical Co. Ltd.). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P. Finkel in SÖFW-Journal 122, 8/1996, S. 543-548 sowie Parf. Kosm. 80. Jahrgang, Nr. 3/1999, S. 10 bis 16 zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. alpha-Carotin, beta-Carotin, Lycopin) und deren Derivate, Chlorogensäure und deren Derivate, Liponsäure und deren Derivate (z.B. Dihydroliponsäure), Auro-thioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl- , Amyl-, Butyl- und Lauryl-, Palmitoyl-, Oleyl-, -Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis mol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascor-bylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO4) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

In einer Ausführungsform der Erfindung enthaltend die erfindungsgemäßen Konzentrate bzw. Zubereitungen als weiteren Bestandteil mindestens einen desodorierenden Wirkstoff.

Desodorierende Wirkstoffe wirken Körpergerüchen entgegen, überdecken oder beseitigen sie. Körpergerüche entstehen durch die Einwirkung von Hautbakterien auf apokrinen Schweiss, wobei unangenehm riechende Abbauprodukte gebildet werden. Dementsprechend eignen sich als deosodorierende Wirkstoffe u.a. keimhemmende Mittel, Enzyminhibitoren, Geruchsabsorber oder Geruchsüberdecker.

Als Stabilisatoren können Metallsalze von Fettsäuren, wie z. B. Magnesium-, Aluminium - und/oder Zinkstearat bzw. -ricinoleat eingesetzt werden.

Die weiteren Bestandteile der Konzentrate bzw. Zubereitungen (wie z.B. Konservierungsmittel, kosmetische Wirkstoffe wie z.B. DHA - Dihydroxyaceton -, UV-Filter etc.) werden je nach ihrer Löslichkeit entweder über die Wasserphase oder über die lipophile Phase zugegeben.

Die wässrige Phase, mit der das Emulsionskonzentrat verdünnt wird, bzw. die wässrige Phase der erfindungsgemäßen kosmetischen oder pharmazeutischen Zubereitungen kann verschiedene wasserlösliche Bestandteile, z.B. wasserlösliche kosmetische Wirkstoffe, wasserlösliche Proteine oder Proteinabbauprodukte, Konservierungsmittel, Farbstoffe, Duftstoffe, Magnesiumsalze oder andere übliche wasserlösliche Komponenten gelöst enthalten. Bevorzugt enthält die wässrige, kontinuierliche Phase ein wasserlösliches, natürliches oder synthetisches Polymerisat, das die kosmetischen Eigenschaften der Emulsionen durch Erhöhung der Viskosität verbessert. Eine besonders wirksame Kombination von Hydrocolloiden zur Verbesserung der kosmetischen Eigenschaften solcher Emulsionen ist ein Gemisch aus nichtionischen Celluloseethem, z.B. Hydroxypropylcellulose und vernetzten Acrylsäure-Polymerisaten, wie sie z.B. unter der Handelsbezeichnung Carbopol® erhältlich sind (vgl. DE 3521713 A1). Darüber hinaus sind besonders geeignete Polymere unter den Handelsbezeichnungen (Cosmedia^{®} ATH, Cosmedia^{®} SP, Cosmedia^{®} CHT (E), Ultragel™ 300, Rheocare™ TTA, Rheocare™ TTN, Rheocare™ TTN-2, Cosmedia®Triple C, Rheocare™C plus) bekannt.

### Verwendung zur Behandlung von Substraten und behandelte Substrate

Die erfindungsgemäßen Emulsionskonzentrate sowie die erfindungsgemäßen kosmetischen oder pharmazeutischen Zubereitungen eignen sich insbesondere zum Behandeln von Substraten.

Ein weiter Gegenstand der Erfindung ist daher die Verwendung der erfindungsgemäßen Emulsionskonzentrate oder der erfindungsgemäßen Zubereitungen zur Behandlung von Substraten, insbesondere von Papieren, Vliesen (Nonwoven) und Geweben (Woven). Erfindungsgemäß zählen hierzu alle Papierarten, Vliese und Gewebe, die dem Fachmann geläufig sind, und Produkte, die daraus herstellbar sind, wie z. B. Toilettenpapier, Papiertaschentücher, Tissues, Wipes, Watte, Wattepads, Make-up Entferner, Tampons, Binden, SlipEinlagen, Windeln, Babypflegetücher, Babyreinigungstücher, Textilien, etc.

Der Begriff **Behandeln** umfasst dabei jede Art der Aufbringung eines erfindungsgemäßen Emulsionskonzentrats sowie einer erfindungsgemäßen kosmetischen oder pharmazeutischen Zubereitungen auf mindestens eine Seite des Substrats. Hierzu eignen sich alle einschlägig bekannten Methoden, mit deren Hilfe man Flüssigkeiten auf mehr oder weniger feste Oberflächen auftragen kann. Beispielsweise seien genannt: Imprägnieren, Tränken, Beschichten, Be- oder Ansprühen, Tauchen, Ausrüsten, Abstreifen etc. Das Behandeln kann dabei bei Raumtemperatur oder unter Hitzeeinwirkung vorgenommen werden. Nach dem Auftragen der Zusammensetzungen kann sich ein kurzer Trockenschritt anschließen.

Die Emulsionskonzentrate sowie die Zubereitungen sind besonders geeignet zur Applikation auf Substraten, wie beispielsweise Papieren, Tüchern, Textilien und Watteprodukten, die im Bereich der Babypflege und -hygiene sowie im Bereich der Make-up-Entfernung, insbesondere der Augen-Make-up-Entfernung, im Bereich der Damenhygiene (Tampons, Damenbinden, Slip-Einlagen) und im Bereich der Körperhygiene (Toilettenpapier, Feuchttoilettenpapier) eingesetzt werden.

Ein weiterer Gegenstand der Erfindung betrifft ein Substrat, welches mindestens folgende Bestandteile enthält:
(A) Alkyl- und/oder Alkenyloligoglykosid(e) mit 6 bis 22 Kohlenstoffatomen im Alk(en)ylrest,
(B) Polyglycerolester,
(C) Polyol(e),
(D) Glycerid(e), die einen Schmelzpunkt größer gleich 20 °C aufweisen
(E) bei 20 °C flüssige Ölkomponente(n) mit einer Polarität von größer 30 - 40 mN/m und
(F) bei 20 °C flüssige Ölkomponente(n) mit einer Polarität von 5 - 30 mN/m,
wobei
- die Komponenten (A), (B) und (C) ein Gewichtsverhältnis A : B : C von 1 : (0,6 - 0,8) : (0,9 - 1,1) aufweisen,
- die Komponenten (A) und (D) ein Gewichtsverhältnis A : D von 4 : 1 bis 1,5 : 1 aufweisen,
- die Komponenten (E) und (F) ein Gewichtsverhältnis E : F von 1 : 1 bis 1 : 3 aufweisen, und

Die erfindungsgemäßen **Substrat** sind beispielsweise erhältlich, indem man ein erfindungsgemäßes Emulsionskonzentrat oder eine erfindungsgemäße Zubereitung auf ein Substrat aufträgt.

Die erfindungsgemäßen Substrate können beispielsweise als Pflege - oder Reinigungstücher eingesetzt werden. Dabei sind sowohl Anwendungen im Bereich der Hautpflege- oder reinigung (insbesondere der Babypflege- oder reinigung) möglich. Beispielsweise seien genannt Pflege- oder Reinigungstücher für die Gesichtshaut (sog. Facial tissues, Abschminktücher/Make-up Entfernung etc), Erfrischungstücher für die Haut, antibakterielle und/oder desodorierende Tücher, Produkte für die Intimpflege; wie beispielsweise Tampons, Damenbinden, Slipeinlagen, Intimpflegetücher), trockenes oder feuchtes Toilettenpapier, Inkontinenzprodukte, Selbstbräunungstücher oder sog. Insect Repellent Tücher. Mit den erfindungsgemäßen Zusammensetzungen ist es möglich die für die jeweilige Anwendung erforderlichen Bestandteile (z.B. desodorierende Wirkstoffe oder öllösliche Pflegekomponenten) auf das Substrat aufzubringen. Die so behandelten Substrate eignen sich weiterhin zur Desinfektion von Haut und Haar.

Ein weiterer Gegenstand der Erfindung betrifft daher die Verwendung eines erfindungsgemäßen Substrats zur Reinigung und/oder Pflege von Haut und Haar.

Ein weiterer Gegenstand der Erfindung betrifft daher die Verwendung eines erfindungsgemäßen Substrats zur Desinfektion von Haut und Haar.

Als Substrat eignet sich prinzipiell jeglicher Träger, der es erlaubt die Zusammensetzung bzw. ihre Bestandteile aufzunehmen. Als **Substrat** geeignet sind beispielsweise Tissuepapiere und/oder Tissuegewebe und/oder Tissuetücher (im Weiteren mit Tissuetücher bezeichnet). Diese können ein- oder mehrlagig aufgebaut sein. In der Regel weisen die Papiere ein Quadratmetergewicht von 10 bis 65, vorzugsweise 15 bis 30 g und eine Dichte von 0,6 g/cm und weniger auf. Beispiele für Tissuepapiere sind Toilettenpapiere, Papiertaschentücher, Gesichtsreinigungstücher, Abschminktücher, Erfrischungstücher, Haushaltstücher und dergleichen. Neben den papierbasierten Tissues kommen auch entsprechende Tissuegewebe in Frage, die aus Faser- oder Fleecestoff hergestellt werden.

Erfindungsgemäß als Substrat bevorzugt sind mehrlagige Tissuetücher. Insbesondere sind solche Tissuetücher bevorzugt, welche zwischen den einzelnen Lagen eine undurchlässige und/oder teildurchlässige Sperrschicht haben. Die teildurchlässige Sperrschicht kann beispielsweise als semipermeable Membran ausgebildet sein. Mit derartigen Tüchern können zwei oder mehrere Emulsionskonzentrate (gegebenenfalls nach vorheriger Verdünnung) auf ein Tuch aufgebracht werden. Dies kann ganz besonders bevorzugt sein, um mit der einen Seite der Tücher die Reinigung mittels der auf das Tuch aufgebrachten Zusammensetzung zu bewirken. Mit der anderen Seite kann dann beispielsweise zum Trocknen nach gerieben werden oder gegebenenfalls ein pflegender Wirkstoff auf die Haut aufgetragen werden.

Weiterhin kann es erfindungsgemäß ganz besonders bevorzugt sein, wenn die Tücher aus mindestens 3 Lagen mit Emulsionskonzentraten (gegebenenfalls nach vorheriger Verdünnung) behandelten Tissuetuches bestehen. Vorteilhaft ist dann zwischen mindestens 2 Lagen behandeltem Tuch jeweils 1 Lage Tuch als semipermeable Membran ausgebildet. Die semipermeable Membran ist dabei in Richtung der äußeren Tuchlagen durchlässig. Dadurch kann im Inneren beispielsweise ein Emulsionskonzentrat (gegebenenfalls nach vorheriger Verdünnung) auf die innerste Schicht aufgebracht werden, welche entweder nicht mischbar und/oder nicht stabil mit dem auf die äußere Seite aufgetragenen Emulsionskonzentrat ist. Hierdurch wird es möglich "two in one Tücher" zur Reinigung und Pflege anzubieten. Selbstverständlich umfasst die Erfindung die unterschiedliche farbliche Gestaltung der Tuchlagen. Weiterhin umfasst die erfindungsgemäße Lehre auch den Aufbau der Tücher aus mehreren Materialien, insbesondere in Bezug auf die Saugfähigkeit und Durchlässigkeit der unterschiedlichen Tuchlagen.

Als Substrat eignen sich weiterhin beispielsweise Textilfasern sowohl aus Naturfasern wie z.B. Cellulose, Seide, Wolle, Regeneratcellulose (Viskose, Rayon) Cellulosederivate als auch Textilfasern aus synthetischen Fasern wie z.B. Polyester-, Polypropylen-, Polyethylenterephtalat-, Polyamid-, Polyolefin-, Polyacrylnitril-Fasern oder Mischungen solcher Fasern. Dieser Fasern können gewebt oder ungewebt sein.

Die erfindungsgemäßen Substrate können nach dem Fachmann bekannten Verfahren hergestellt werden. Der Auftrag der erfindungsgemäßen Emulsionskonzentrate oder der erfindungsgemäßen Zubereitungen erfolgt durch Behandeln der Substrate nach dem Fachmann bekannten Methoden.

Die erfindungsgemäßen Emulsionskonzentrate oder die erfindungsgemäßen Zubereitungen können vor dem Behandeln der Substrate verdünnt werden und gegebenenfalls kann das erhaltene Substrat anschließend getrocknet werden.

### Beispiele

Die folgenden Beispiele sollen die vorliegende Erfindung näher erläutern.

Die in Tab. 1 aufgeführten Emulsionskonzentrate wurden wie folgt hergestellt.

Alkyl- und/oder Alkenyloligoglykoside (A), Polyglycerolester (B) zusammen mit dem Polyol (C), Glycerid (D), öllöslichen Komponenten (E) und (F) und Wasser wurden unter Rühren auf 70 - 75°C für 60 min. erhitzt. Anschließend wurde unter Rühren auf 30°C abgekühlt und ggf. der pH-Wert auf pH 5,0 - 6,0 eingestellt.

Tabelle 1 zeigt die Zusammensetzung der Emulsionskonzentrate und deren Viskositäten. Die Viskosität wurde sowohl bei Raumtemperatur (23°C) als auch bei 15°C mit einem Brookfield Rotationsviskosimeter (Typ RVF, Spindel 5, 10 UpM) bestimmt. Es zeigte sich, dass sowohl eine gute Lagerstabilität als auch gute Viskositätseigenschaften, sowohl bei Raumtemperatur als auch bei niedriger Temperatur, bei Einhaltung der erfindungsgemäßen Parameter, erhalten wurden.

Des Weiteren sind die Stabilitätsergebnisse der Verdünnungen mit Wasser sowie deren Teilchengröße dargestellt. Die erfindungsgemäßen Verdünnungen der Emulsionskonzentrate sind bei Lagerung stabil.

**Tab. 1: Zusammensetzung und Ergebnisse erfindungsgemäßer Emulsionskonzentrate (alle Angaben in Gew.-% bezogen auf das Gewicht des Konzentrats)**

| | **Komponente INCI** | **Bsp. 1** | **Bsp. 2** | **Bsp. 3** | **Bsp. 4** |
|---|---|---|---|---|---|
| (A) | Lauryl Glucoside | 15,0 | 13,75 | 16,25 | 13,75 |
| (B) | Polyglyceryl-2-Dipolyhydroxystearat | 11,25 | 10,35 | 12,15 | 10,35 |
| (C) | Glycerin | 15,0 | 13,75 | 16,25 | 13,75 |
| (D) | Glyceryl-polyhydroxystearate | 3,75 | 3,4 | 4,1 | 3,4 |
| (D) | Glyceryl Oleate | 3,0 | 3,0 | 3,0 | 3,0 |
| (E) | Dicaprylyl Ether | 13,0 | 13,0 | - | - |
| | Cetearyl Alcohol | - | - | - | - |
| (E) | Dicaprylyl Carbonate | - | - | 8,0 | - |
| (E) | Propylheptyl Caprylate | - | - | - | 13,0 |
| (F) | Caprylic/Capric Triglyceride | 24,0 | 29,0 | 24,0 | 29,0 |
| (F) | Olus | - | - | - | - |
| | Citric Acid (zur PH-Einstellung) | | | | |
| | Wasser | 15,0 | 13,75 | 16,25 | 13,75 |
| | Viskosität (RT), mPa*s | 3600 | 2000 | 6400 | 1200 |
| | Viskosität (15°C), mPa*s | 6400 | 4800 | 14000 | 10000 |
| | Teilchengröße, 5% Verdünnung Mittelwert (Coulter LS), nm | 81 | 82 | 79 | 83 |
| | Aussehen 5 % wässrige Verdünnung | transparent | transparent | transparent. | Transparent |

**Tabelle 2 zeigt die Verhältnisse der Komponenten zueinander.**

| **Beispiel** | **Verhältnis (A) : (B) : (C)** | **Verhältnis (A) : (D)** | **Verhältnis (E) : (F)** |
|---|---|---|---|
| 1 | 1 : 0,75 : 1 | 2,2 : 1 | 1 : 1,3 |
| 2 | 1 : 0,75 : 1 | 2,1 : 1 | 1 : 2,2 |
| 3 | 1 : 0,75 : 1 | 2,3 : 1 | 1 : 3 |
| 4 | 1 : 0,75 : 1 | 2,1 : 1 | 1 : 2,2 |

**Tab 5: Emulsionskonzentrat - wässrige Verdünnungen. Alle Angaben in Gew.% Aktivsubstanz bezogen auf das Gesamtgewicht der Zubereitung.**

| **Komponente** | **1** | **2** | **3** | **4** | **5** | **6** | **7** | **8** | **9** |
|---|---|---|---|---|---|---|---|---|---|
| | | | | | | | | | |
| Emulsionskonzentrat qemäß Bsp. 1* | 5.0 | 4.0 | 7.5 | 3.5 | 6.5 | 2.0 | 7.5 | 9.0 | 5.5 |
| Euxyl^{®} K 300 | | | | 0.8 | | | 0.8 | | |
| Nipaquard^{®}BPX | | | 0.5 | | | | | | |
| Sodium Benzoate | 0.5 | | | | | | | | 0.5 |
| Potassium Sorbate | | | | | | | | | 0.2 |
| EuxyIPE^{®}9010 | | | | | | 1.0 | | | |
| Nipaguard^{®}P05 | | 1.0 | | | 1.0 | | | 1.0 | |
| Sensiva SC®10 | 0.2 | | | | | 0.1 | | | |
| Herbalia^{®}Green Tea (Camellia sinensis and Silica) | | | | | | 0.05 | | | |
| Cosmedia^{®}SP | | 0.3 | | 0.2 | | | | | |
| Melhydran^{®} LS 4420 | | | 0.5 | | | | | | |
| Lactolan^{®} LS 5879 | | | 0.5 | | | | | | |
| Copherol^{®} 1250 C | | | | | 0.4 | | | | |
| Dihydroxyacetone (DHA) | | | | | | | | | 2.0 |
| Insect Repellent 3535^{®} | | | | | | | | 10.0 | |
| Eusolex^{®} OCR | | | | | | | 1.0 | | |
| Eusolex^{®} 9020 | | | | | | | 0.3 | | |
| Myritol^{®} 318 | | | | 0.1 | | | | | |
| Parfüm | | 0.1 | 0.2 | | 0.3 | | 0.1 | | 0.25 |
| Wasser | add 100 | | | | | | | | |
| pH-Wert der Verdünnung (ggf. mit pH-Adjuster einstellen) | 4.2 - 5.0 | 6.0 - 7.0 | 4.2 - 5.0 | 6.0 - 6.5 | 6.0 - 6.5 | 6.0 - 6.5 | 6.0 - 6.5 | 6.0 - 6.5 | 3.5 - 4.0 |

| | | | | | | | | | |
|---|---|---|---|---|---|---|---|---|---|
| * Alternativ können anstelle des Emulsionskonzentrats nach Bsp. 1 auch die von Bsp. 2, Bsp.3 oder Bsp. 4 verwendet werden. Verwendete Handelsprodukte: Euxyl^{®}K 300 INCI: Phenoxyethanol, Methylparaben, Ethylparaben, Propylparaben, Butylparaben, Isobutylparaben; Nipaguard^{®}BPX INCI: Phenoxyethanol, Methylparaben, Propylparaben, 2-Bromo-2-Nitro-propane-1,3-Diol; EuxyIPE^{®}9010 INCI: Phenoxyethanol, Ethylhexylglycerin; Nipaguard^{®}P05 INCI: Phenoxyethanol, Piroctone Olamine; Sensiva® SC 10 INCI: Caprylyl Glycol, Ethylhexylglycerin; Cosmedia^{®}SP INCI: Sodium Polyacrylate; Melhydran^{®} LS 4420 INCI: Honey extract; Lactolan^{®} LS 5879 INCI: Hydrolyzed Milk Protein; Copherol^{®} 1250 C INCI: Tocopheryl Acetate; Insect Repellent 3535^{®} INCI: Ethyl Butylacetylaminopropionate; Eusolex^{®} OCR INCI: Octocrylene; Eusolex^{®} 9020 INCI: Butyl Methoxydibenzoylmethane; Myritol^{®} 318 INCI: Caprylic/Capric Triglyceride; | | | | | | | | | |

## Patentansprüche

1. Emulsionskonzentrat, enthaltend folgende Komponenten:
(A) Alkyl- und/oder Alkenyloligoglykosid(e) mit 6 bis 22 Kohlenstoffatomen im Alk(en)ylrest,
(B) Polyglycerolester,
(C) Polyol(e),
(D) Glycerid(e), die einen Schmelzpunkt größer gleich 20 °C aufweisens
(E) bei 20 °C flüssige Ölkomponente(n) mit einer Polarität von größer 30 - 40 mN/m und
(F) bei 20 °C flüssige Ölkomponente(n) mit einer Polarität von 5 - 30 mN/m,
wobei
- die Komponenten (A), (B) und (C) ein Gewichtsverhältnis A : B : C von 1 : (0,6 - 0,8) : (0,9 - 1,1) aufweisen,
- die Komponenten (A) und (D) ein Gewichtsverhältnis A : D von 4 : 1 bis 1,5 : 1 aufweisen,
- die Komponenten (E) und (F) ein Gewichtsverhältnis E : F von 1 : 1 bis 1 : 3 aufweisen, und
- der Wasseranteil kleiner gleich 20 Gew.-% bezogen auf das Gesamtgewicht des Emulsionskonzentrats beträgt.

2. Emulsionskonzentrat nach Anspruch 1, **dadurch gekennzeichnet, dass** als Alkyl- und/oder Alkenyloligoglycosid(e) die Verbindungen der allgemeinen Formel (I) eingesetzt werden
Gₘ-R¹ (I)
- in der G für einen Zuckerrest mit 5 oder 6 C Atomen steht,
- R¹ für einen C6 bis C22 Alkyl- und/oder Alkenylrest in Acetalbindung steht,
- m einen mittleren Wert von 1 bis 10, vorzugsweise 1 bis 8, insbesondere 1 bis 3, vorzugsweise 1,2 bis 1,8 darstellt.

3. Emulsionskonzentrat nach Anspruch 1 oder 2,
welches als Komponente (B) einen Polyglycerolester einer Polyhydroxystearinsäure aufweist.

4. Emulsionskonzentrat nach einem der vorhergehenden Ansprüche,
welches als Komponente (C) ein Polyol mit 2 bis 6 Hydroxylgruppen aufweist.

5. Emulsionskonzentrat nach Anspruch 4, **dadurch gekennzeichnet, dass** die Polyole 2 bis 18 C-Atome enthalten.

6. Emulsionskonzentrat nach einem der vorhergehenden Ansprüche, welches als Komponente (D) Glyceride von C18 Fettsäuren enthält, vorzugsweise Glyceride von C18 Fettsäuren, worin der Anteil an Glycerinmonoester größer gleich 90 Gew.-%, bezogen auf das Gewicht aller Glyceride von C18 Fettsäuren, beträgt.

7. Emulsionskonzentrat nach einem der vorhergehenden Ansprüche, **dadurch gekennzeichnet, dass** es weniger als 10 Gew.-%, vorzugsweise weniger als 5 Gew.-% ethoxylierten Substanzen enthält.

8. Emulsionskonzentrat nach einem der vorhergehenden Ansprüche, **dadurch gekennzeichnet, dass** es eine Viskosität bei 15°C von unterhalb 30000 mPa*s aufweist.

9. Verwendung des Emulsionskonzentrats nach einem der Ansprüche 1 bis 8 als kosmetische oder pharmazeutische Zubereitung.

10. Verwendung des Emulsionskonzentrats nach einem der Ansprüche 1 bis 8 zur Herstellung kosmetischer oder pharmazeutischer Zubereitungen, insbesondere Öl-in-Wasser-Emulsionen.

11. Kosmetische oder pharmazeutische Zubereitung enthaltend folgende Komponenten:
(A) Alkyl- und/oder Alkenyloligoglykosid(e) mit 6 bis 22 Kohlenstoffatomen im Alk(en)ylrest,
(B) Polyglycerolester,
(C) Polyol(e),
(D) Glycerid(e), die einen Schmelzpunkt größer gleich 20 °C aufweisen,
(E) bei 20 °C flüssige Ölkomponente(n) mit einer Polarität von größer 30 - 40 mN/m und
(F) bei 20 °C flüssige Ölkomponente(n) mit einer Polarität von 5 - 30 mN/m,
wobei
- die Komponenten (A), (B) und (C) ein Gewichtsverhältnis A : B : C von 1 : (0,6 - 0,8) : (0,9 - 1,1) aufweisen,
- die Komponenten (A) und (D) ein Gewichtsverhältnis A : D von 4 : 1 bis 1,5 : 1 aufweisen,
- die Komponenten (E) und (F) ein Gewichtsverhältnis E : F von 1 : 1 bis 1 : 3 aufweisen, und
- der Wasseranteil kleiner gleich 20 Gew.-% bezogen auf das Gesamtgewicht des Emulsionskonzentrats beträgt.

12. Verwendung der Emulsionskonzentrate nach einem der Ansprüche 1 bis 8 oder der kosmetischen oder pharmazeutischen Zubereitungen nach Anspruch 11 zur Behandlung von Substraten.

13. Substrat, insbesondere Papier-, Vlies- oder Gewebe-Produkt, welches mindestens folgende Bestandteile enthält:
(A) Alkyl- und/oder Alkenyloligoglykosid(e) mit 6 bis 22 Kohlenstoffatomen im Alk(en)ylrest,
(B) Polyglycerolester,
(C) Polyol(e),
(D) Glycerid(e), die einen Schmelzpunkt größer gleich 20 °C aufweisen,
(E) bei 20 °C flüssige Ölkomponente(n) mit einer Polarität von größer 30 - 40 mN/m und
(F) bei 20 °C flüssige Ölkomponente(n) mit einer Polarität von 5 - 30 mN/m,
wobei
- die Komponenten (A), (B) und (C) ein Gewichtsverhältnis A : B : C von 1 : (0,6 - 0,8) : (0,9 - 1,1) aufweisen,
- die Komponenten (A) und (D) ein Gewichtsverhältnis A : D von 4 : 1 bis 1,5 : 1 aufweisen,
- die Komponenten (E) und (F) ein Gewichtsverhältnis E : F von 1 : 1 bis 1 : 3 aufweisen, und
- der Wasseranteil kleiner gleich 20 Gew.-% bezogen auf das Gesamtgewicht des Emulsionskonzentrats beträgt.

14. Verwendung eines Substrates nach Anspruch 13 zur Körperpflege und/oder Reinigung.

## Claims

1. Emulsion concentrate comprising the following components:
(A) alkyl and/or alkenyl oligoglycoside(s) having 6 to 22 carbon atoms in the alk(en)yl radical,
(B) polyglycerol ester,
(C) polyol(s),
(D) glyceride(s) which have a melting point greater than or equal to 20°C,
(E) oil component (s) liquid at 20°C and with a polarity of more than 30-40 mN/m and
(F) oil component (s) liquid at 20°C and with a polarity of 5-30 mN/m,
where
- the components (A), (B) and (C) have a weight ratio A:B:C of 1:(0.6-0.8):(0.9-1.1),
- the components (A) and (D) have a weight ratio A:D of from 4:1 to 1.5:1,
- the components (E) and (F) have a weight ratio E:F of from 1:1 to 1:3, and
- the water fraction is less than or equal to 20% by weight, based on the total weight of the emulsion concentrate.

2. Emulsion concentrate according to Claim 1, **characterized in that** the alkyl and/or alkenyl oligoglycoside(s) used are the compounds of the general formula (I)
Gₘ-R¹ (I)
- in which G is a sugar radical having 5 or 6 carbon atoms,
- R¹ is a C6 to C22 alkyl and/or alkenyl radical in acetal bond,
- m is an average value from 1 to 10, preferably 1 to 8, in particular 1 to 3, preferably 1.2 to 1.8.

3. Emulsion concentrate according to Claim 1 or 2, which has a polyglycerol ester of a polyhydroxystearic acid as component (B).

4. Emulsion concentrate according to any one of the preceding claims, which has a polyol having 2 to 6 hydroxyl groups as component (C).

5. Emulsion concentrate according to Claim 4, **characterized in that** the polyols contain 2 to 18 carbon atoms.

6. Emulsion concentrate according to any one of the preceding claims, which comprises, as component (D), glycerides of C18 fatty acids, preferably glycerides of C18 fatty acids in which the fraction of glycerol monoester is greater than or equal to 90% by weight, based on the weight of all of the glycerides of C18 fatty acids.

7. Emulsion concentrate according to any one of the preceding claims, **characterized in that** it comprises less than 10% by weight, preferably less than 5% by weight, of ethoxylated substances.

8. Emulsion concentrate according to any one of the preceding claims, **characterized in that** it has a viscosity at 15°C of below 30 000 mPa*s.

9. Use of the emulsion concentrate according to any one of Claims 1 to 8 as cosmetic or pharmaceutical preparation.

10. Use of the emulsion concentrate according to any one of Claims 1 to 8 for producing cosmetic or pharmaceutical preparations, in particular oil-in-water emulsions.

11. Cosmetic or pharmaceutical preparation comprising the following components:
(A) alkyl and/or alkenyl oligoglycoside(s) having 6 to 22 carbon atoms in the alk(en)yl radical,
(B) polyglycerol ester,
(C) polyol(s),
(D) glyceride(s) which have a melting point greater than or equal to 20°C,
(E) oil component(s) liquid at 20°C and with a polarity of more than 30-40 mN/m and
(F) oil component(s) liquid at 20°C and with a polarity of 5-30 mN/m,
where
- the components (A), (B) and (C) have a weight ratio A:B:C of 1:(0.6-0.8):(0.9-1.1),
- the components (A) and (D) have a weight ratio A:D of from 4:1 to 1.5:1,
- the components (E) and (F) have a weight ratio E:F of from 1:1 to 1:3, and
- the water fraction is less than or equal to 20% by weight, based on the total weight of the emulsion concentrate.

12. Use of the emulsion concentrates according to any one of Claims 1 to 8 or of the cosmetic or pharmaceutical preparations according to Claim 11 for treating substrates.

13. Substrate, in particular paper, nonwoven or woven product, which comprises at least the following constituents:
(A) alkyl and/or alkenyl oligoglycoside(s) having 6 to 22 carbon atoms in the alk(en)yl radical,
(B) polyglycerol ester,
(C) polyol(s),
(D) glyceride(s) which have a melting point greater than or equal to 20°C,
(E) oil component(s) liquid at 20°C and with a polarity of more than 30-40 mN/m and
(F) oil component(s) liquid at 20°C and with a polarity of 5-30 mN/m,
where
- the components (A), (B) and (C) have a weight ratio A:B:C of 1:(0.6-0.8):(0.9-1.1),
- the components (A) and (D) have a weight ratio A:D of from 4:1 to 1.5:1,
- the components (E) and (F) have a weight ratio E:F of from 1:1 to 1:3, and
- the water fraction is less than or equal to 20% by weight, based on the total weight of the emulsion concentrate.

14. Use of a substrate according to Claim 13 for bodycare and/or cleaning.

## Revendications

1. Concentré d'émulsion, contenant les composants suivantes :
(A) un/des alkyl- et/ou alcényloligoglycoside(s) ayant de 6 à 22 atomes de carbone dans le radical alkyle/ alcényle,
(B) un ester de polyglycérol,
(C) un/des polyol(s),
(D) un/des glycéride(s) qui présente(nt) un point de fusion supérieur ou égal à 20 °C,
(E) un/des composant(s) huileux liquide(s) à 20 °C, ayant une polarité supérieure à 30 - 40 mN/m et
(F) un/des composant(s) huileux liquide(s) à 20 °C, ayant une polarité de 5 - 30 mN/m,
- les composants (A), (B) et (C) ayant un rapport pondéral A : B : C de 1 : (0, 6 - 0,8) : (0, 9 - 1,1),
- les composants (A) et (D) ayant un rapport pondéral A : D de 4 : 1 à 1,5 : 1,
- les composants (E) et (F) ayant un rapport pondéral E : F de 1 : 1 à 1 : 3, et
- la teneur en eau étant inférieure ou égale à 20 % en poids, par rapport au poids total du concentré d'émulsion.

2. Concentré d'émulsion selon la revendication 1, **caractérisé en ce qu'**on utilise comme alkyl- et/ou alcényloligoglycoside(s) les composés de formule générale (I)
Gₘ-R¹ (I)
- dans laquelle G représente un radical glucidique ayant 5 ou 6 atomes de carbone,
- R¹ représente un radical alkyle et/ou alcényle en C₆-C₂₂ en liaison acétal,
- m représente une valeur moyenne de 1 à 10, de préférence de 1 à 8, en particulier de 1 à 3, de préférence de 1,2 à 1,8.

3. Concentré d'émulsion selon la revendication 1 ou 2, qui comporte comme composant (B) un ester de polyglycérol d'un acide polyhydroxystéarique.

4. Concentré d'émulsion selon l'une quelconque des revendications précédentes, qui comporte comme composant (C) un polyol comportant de 2 à 6 groupes hydroxy.

5. Concentré d'émulsion selon la revendication 4, **caractérisé en ce que** les polyols contiennent de 2 à 18 atomes de carbone.

6. Concentré d'émulsion selon l'une quelconque des revendications précédentes, qui contient comme composant (D) des glycérides d'acides gras en C18, de préférence des glycérides d'acides gras en C18, dans lesquels la proportion de monoester de glycérol est supérieure ou égale à 90 % en poids, par rapport au poids de tous les glycérides d'acides gras en C18.

7. Concentré d'émulsion selon l'une quelconque des revendications précédentes, **caractérisé en ce qu'**il contient moins de 10 % en poids, de préférence moins de 5 % en poids de substances éthoxylées.

8. Concentré d'émulsion selon l'une quelconque des revendications précédentes, **caractérisé en ce qu'**il présente une viscosité à 15 °C inférieure à 30 000 mPa.s.

9. Utilisation du concentré d'émulsion selon l'une quelconque des revendications 1 à 8, en tant que préparation cosmétique ou pharmaceutique.

10. Utilisation du concentré d'émulsion selon l'une quelconque des revendications 1 à 8, pour la fabrication de préparations cosmétiques ou pharmaceutiques, en particulier d'émulsions huile-dans-eau.

11. Préparation cosmétique ou pharmaceutique contenant les composants suivantes :
(A) un/des alkyl- et/ou alcényloligoglycoside(s) ayant de 6 à 22 atomes de carbone dans le radical alkyl/alcényle,
(B) un ester de polyglycérol,
(C) un/des polyol(s),
(D) un/des glycéride(s) qui présente(nt) un point de fusion supérieur ou égal à 20 °C,
(E) un/des composant(s) huileux liquide(s) à 20 °C, ayant une polarité supérieure à 30 - 40 mN/m et
(F) un/des composant(s) huileux liquide(s) à 20 °C, ayant une polarité de 5 - 30 mN/m,
- les composants (A), (B) et (C) ayant un rapport pondéral A : B : C de 1 : (0, 6 - 0,8) : (0, 9 - 1,1),
- les composants (A) et (D) ayant un rapport pondéral A : D de 4 : 1 à 1,5 : 1,
- les composants (E) et (F) ayant un rapport pondéral E : F de 1 : 1 à 1 : 3, et
- la teneur en eau étant inférieure ou égale à 20 % en poids, par rapport au poids total du concentré d'émulsion.

12. Utilisation des concentrés d'émulsions selon l'une quelconque des revendications 1 à 8 ou des préparations cosmétiques ou pharmaceutiques selon la revendication 11, pour le traitement de substrats.

13. Substrat, en particulier produit en papier, non-tissé ou tissu, qui contient au moins les composants suivantes :
(A) un/des alkyl- et/ou alcényloligoglycoside(s) ayant de 6 à 22 atomes de carbone dans le radical alkyl/alcényle,
(B) un ester de polyglycérol,
(C) un/des polyol(s),
(D) un/des glycéride(s) qui présente(nt) un point de fusion supérieur ou égal à 20 °C,
(E) un/des composant(s) huileux liquide(s) à 20 °C, ayant une polarité supérieure à 30 - 40 mN/m et
(F) un/des composant(s) huileux liquide(s) à 20 °C, ayant une polarité de 5 - 30 mN/m,
- les composants (A), (B) et (C) ayant un rapport pondéral A : B : C de 1 : (0, 6 - 0,8) : (0, 9 - 1,1),
- les composants (A) et (D) ayant un rapport pondéral A : D de 4 : 1 à 1,5 : 1,
- les composants (E) et (F) ayant un rapport pondéral E : F de 1 : 1 à 1 : 3, et
- la teneur en eau étant inférieure ou égale à 20 % en poids, par rapport au poids total du concentré d'émulsion.

14. Utilisation d'un substrat selon la revendication 13, pour le nettoyage et/ou le soin du corps.
